# FASCICULE DE BREVET EUROPEEN

(11) **EP 1 935 400 B1**
(45) Date de publication et mention de la délivrance du brevet: **04.09.2013**
(21) Numéro de dépôt: 07123391.0
(22) Date de dépôt: 17.12.2007
(51) Int. Cl.: A61K 8/81, A61K 8/90, A61K 8/92, A61K 8/97, A61Q 1/02, A61Q 1/04, A61Q 1/06

(54) **Composition de maquillage de la peau comprenant une résine, un copolymère bloc, un corps gras solide, exempte d'huile volatile**
Zusammensetzung zum Schminken der Haut, die ein Harz, ein Blockcopolymer, einen festen Fettstoff und kein flüchtiges Öl enthält
Composition for skin make-up comprising a resin, a block copolymer, a solid fat, and no volatile oils

(30) Priorité: 19.12.2006 FR 0655650; 19.12.2006 FR 0655651
(43) Date de publication de la demande: 25.06.2008
(73) Titulaire: L'Oréal, 75008 Paris (FR)
(72) Inventeur: Jaques, Véronique, 92340, BOURG LA REINE (FR); Schwartz, Véronique, 92290, CHATENAY MALABRY (FR); Blin, Xavier, 75015, PARIS (FR)
(74) Mandataire: Duvert, Sandra

(56) Documents cités:
- EP-A- 0 205 961
- EP-A- 1 044 674
- EP-A- 1 621 229
- EP-A- 1 759 690
- US-B1- 6 303 105

## Description

La présente invention se rapporte à une composition cosmétique selon la revendication 1 pour le maquillage ou le soin de la peau, y compris du cuir chevelu, aussi bien du visage que du corps humain, des lèvres ou des phanères des êtres humains, comme les cheveux, les cils, les sourcils ou les ongles, comprenant une phase grasse contenant une résine, un copolymère bloc hydrocarboné et un corps gras dont la température de fusion est supérieure à 25°C. Cette composition possède des propriétés cosmétiques remarquables et confère en particulier au maquillage ou au soin des propriétés de brillance et de rémanence de la brillance après l'application tout en limitant sa migration.

La composition de l'invention peut en particulier constituer un produit de maquillage du corps, des lèvres ou des phanères d'êtres humains ayant en particulier des propriétés de soin et/ou de traitement non thérapeutique. Elle constitue notamment un rouge à lèvres ou un brillant à lèvres, un fard à joues ou à paupières, un produit pour tatouage, un mascara, un eye-liner, un vernis à ongles, un produit de bronzage artificiel de la peau, un produit de coloration ou de soin des cheveux.

Il existe de nombreuses compositions cosmétiques pour lesquelles les propriétés de brillance et de confort du film déposé, après application sur les matières kératiniques (peau, lèvres, phanères), sont souhaitables. On peut citer par exemple les rouges à lèvres, les vernis à ongles ou encore certains produits capillaires. De plus, il est souhaitable qu'après application, la composition conserve un bon niveau de brillance. La plupart des compositions brillantes ne présentent en effet un bon niveau de brillance que lors de l'application, celui-ci diminuant rapidement après application.

De plus, les compositions contenant des huiles volatiles ont l'inconvénient de laisser sur les lèvres, après évaporation de ces huiles volatiles, un film qui devient très vite inconfortable au cours du temps (sensation de dessèchement, de tiraillement et d'inconfort), écartant un certain nombre de femmes de ce type de rouge à lèvres. De plus, le dépôt obtenu est mat. Or les consommateurs sont toujours à la recherche d'un produit brillant, confortable à porter tout au long de la journée, qui ne migre pas ou peu dans les plis de la peau entourant les lèvres ou les yeux tels que les rides et les ridules.

Le document EP 1621229 A1 décrit des compositions de maquillage des lèvres comprenant une résine hydrocarbonée de poids moléculaire moyen en nombre inférieur ou égal à 10000g/mol associée à des corps gras et des cires.

Le document US 6303105 B1 décrit des compositions de maquillage comprenant des résines de type colophane et des copolymères d'acrylate cyclique d'alkyle.

Le document EP1759690 A2 décrit des compositions pour le maquillage des lèvres comprenant une résine hydrocarbonée de poids moléculaire moyen en nombre inférieur ou égal à 10000g/mol et un polymère bloc styrène/éthylène/butylène.

La présente invention a donc pour but de fournir une nouvelle voie de formulation d'un produit cosmétique qui permette d'obtenir de bonnes propriétés de brillance lors de l'application et après l'application tout en conservant un niveau de migration faible.

Or, la Demanderesse a trouvé de manière surprenante qu'en associant, dans une composition cosmétique de maquillage ou de soin de la peau, une résine particulière, un polymère séquencé hydrocarboné, un corps gras dont la température de fusion est supérieure à 25°C, tel qu'une cire ou un corps gras pâteux en l'absence d'huile volatile (ou en présence d'une faible quantité d'huile volatile), il était possible de réaliser un produit de maquillage présentant un bon niveau de brillance lors de l'application et après l'application.

La présente invention a pour objet une composition cosmétique de maquillage ou de soin de la peau comprenant une phase grasse comprenant :
- au moins une résine de poids moléculaire moyen en nombre inférieur ou égal à 10000 g/mol choisie parmi les résines hydrocarbonées et leurs mélanges,
- au moins un polymère bloc séquencé hydrocarboné selon la revendication 1,
- au moins un corps gras dont la température de fusion est supérieure à 25°C choisi parmi les cires et les corps gras pâteux,
   ladite composition ne contenant pas d'huile volatile ou contenant moins de 5% en poids ou mieux moins de 2% en poids d'huile volatile par rapport au poids total de la composition,
- au moins une huile non volatile, et ladite composition étant sous forme liquide à température ambiente (25°C).

Selon un mode de réalisation avantageux ladite composition comprend au moins une cire et au moins un corps gras pâteux en tant que corps gras dont la température est supérieure à 25°C.

De façon préférée, la composition selon l'invention est anhydre, c'est-à-dire qu'elle est totalement exempte d'eau ou qu'elle en comprend moins de 10% en poids, ou mieux, moins de 4% par rapport au poids total de la composition.

De façon avantageuse, la composition ne comprend pas de composé siliconé, ou en comprend moins de 10% en poids ou mieux moins de 4%, par rapport au poids total de la composition.

La composition est sous forme liquide à température ambiante (25°C).

Un autre avantage de l'invention résolu par certains modes de réalisation est de présenter un collant réduit.

L'invention a également pour objet un procédé non thérapeutique de maquillage ou de soin de la peau comprenant l'application sur la peau d'une composition telle que définie précédemment.

L'invention a encore pour objet l'utilisation d'une composition telle que définie précédemment pour obtenir un dépôt, notamment un maquillage, sur la peau permettant de donner satisfaction en terme de brillance tout en conservant un niveau de migration faible.

### Résine

La résine utilisée dans la composition selon l'invention a de préférence un poids moléculaire moyen en nombre inférieure ou égale à 10 000 g/mol, notamment allant de 250 à 10 000 g/mol de préférence inférieure ou égale à 5 000 g/mol, notamment allant de 250 à 5 000 g/mol, mieux, inférieure ou égale à 2 000 g/mol notamment allant de 250 à 2 000 g/mol et encore mieux inférieure ou égale à 1 000 g/mol notamment allant de 250 à 1 000 g/mol.

On détermine les poids moléculaires moyens en nombre (Mn) par chromatographie liquide par perméation de gel (solvant THF, courbe d'étalonnage établie avec des étalons de polystyrène linéaire, détecteur réfractométrique).

La résine de la composition selon l'invention est avantageusement une résine dite tackifiante. De telles résines sont notamment décrites dans le Handbook of Pressure Sensitive Adhesive, edited by Donatas Satas, 3rd ed., 1989, p. 609-619.

La résine de la composition selon l'invention est choisie parmi les résines hydrocarbonées et leurs mélanges.

Les résines hydrocarbonées sont choisies parmi les polymères de faible poids moléculaire qui peuvent classifiées, selon le type de monomère qu'elles comprennent, en :
- résines hydrocarbonées indéniques telles que les résines issues de la polymérisation en proportion majoritaire de monomère indène et en proportion minoritaire de monomère choisi parmi le styrène, le méthylindène, le méthylstyrène et leurs mélanges. Ces résines pouvant éventuellement être hydrogénées. Ces résines peuvent présenter un poids moléculaire allant de 290 à 1150 g/mol.
   Comme exemples de résines indéniques, on peut citer celles commercialisées sous la référence ESCOREZ 7105 par la société Exxon Chem., NEVCHEM 100 et NEVEX 100 par la société Neville Chem., NORSOLENE S105 par la société Sartomer, PICCO 6100 par la société Hercules et RESINALL par la société Resinall Corp., ou les copolymères indène/méthylstyrène/styrène hydrogéné commercialisées sous la dénomination « REGALITE » par la société Eastman Chemical, en particulier REGALITE R 1100, REGALITE R 1090, REGALITE R-7100, REGALITE R1010 HYDROCARBON RESIN, REGALITE R1125 HYDROCARBON RESIN.
- les résines aliphatiques de pentanediène telles que celle issues de la polymérisation majoritairement du monomères 1,3-pentanediène (trans ou cis pipérylène) et de monomère minoritaire choisi parmi l'isoprène, le butène, le 2-méthyl-2-butène, le pentène, le 1,4-pentanediène et leurs mélanges. Ces résines peuvent présenter un poids moléculaire allant de 1000 à 2500 g/mol.
   De telles résines du 1,3-pentanediène sont commercialisées par exemple sous les références PICCOTAC 95 par la société Eastman Chemical, ESCOREZ 1304 par la société Exxon Chemicals., NEVTAC 100 par la société Neville Chem. ou WINGTACK 95 par la société Goodyear;
- les résines mixtes de pentanediène et d'indène, qui sont issues de la polymérisation d'un mélange de monomères de pentanediène et d'indéne tels que ceux décrits ci-dessus, comme par exemple les résines commercialisées sous la référence ESCOREZ 2101 par la société Exxon Chemicals., NEVPENE 9500 par la société Neville Chem., HERCOTAC 1148 par la société Hercules., NORSOLENE A 100 par la société Sartomer, WINGTACK 86, WINGTACK EXTRA et WINGTACK PLUS par la société Goodyear,
- les résines diènes des dimères du cyclopentanediène telles que celles issues de la polymérisation de premier monomère choisi parmi l'indène et le styrène, et de deuxième monomère choisi parmi les dimères du cyclopentanediène tels que le dicyclopentanediène, le méthyldicyclopentanediène, les autres dimères du pentanediène, et leurs mélanges. Ces résines présentent généralement un poids moléculaire allant de 500 à 800 g/mol, telles que par exemple celles commercialisées sous la référence BETAPRENE BR 100 par la société Arizona Chemical Co., NEVILLE LX-685-125 et NEVILLE LX-1000 par la société Neville Chem., PICCODIENE 2215 par la société Hercules, PETRO-REZ 200 par la société Lawter ou RESINALL 760 par la société Resinall Corp. ;
- les résines diènes des dimères de l'isoprène telles que les résines terpèniques issues de la polymérisation d'au moins un monomère choisi parmi α-pinène, le β-pinène, le limonène, et leurs mélanges. Ces résines peuvent présenter un poids moléculaire allant de 300 à 2000 g/mol. De telles résines sont commercialisées par exemple sous la dénomination PICCOLYTE A115 et S125 par la société Hercules, ZONAREZ 7100 ou ZONATAC 105 LITE par la société ARIZONA Chem.
   On peut également citer certaines résines modifiées telles que les résines hydrogénées comme celles commercialisées sous la dénomination EASTOTAC C6-C20 POLYOLEFINE par la société Eastman Chemical Co., sous la référence ESCOREZ 5300 par la société Exxon Chemicals ou encore les résines NEVILLAC HARD ou NEVROZ proposées par la société Neville Chem., les résines PICCOFYN A-100, PICCOTEX 100 ou PICCOVAR AP25 proposées par la société Hercules ou la résine SP-553 proposée par société Schenectady Chemical Co.

Selon un mode préféré de réalisation, la résine est choisie parmi résines hydrocarbonées indéniques en particulier les copolymères indène/méthylstyrène/styrène hydrogéné commercialisées sous la dénomination « REGALITE » par la société Eastman Chemical, tels que la REGALITE R 1100, REGALITE R 1090, REGALITE R-7100, REGALITE R1010 HYDROCARBON RESIN, REGALITE R1125 HYDROCARBON RESIN.

La résine peut être présente dans la composition selon l'invention en une teneur allant de 0,1 à 30 % en poids, par rapport au poids total de la composition, de préférence allant de 0,3 à 20 % en poids, plus préférentiellement allant de 0,5 à 15 % en poids.

### Copolymère bloc hydrocarboné

La composition comprends, outre la résine, un copolymère séquencé hydrocarboné appelé également copolymère bloc, de préférence un copolymère séquencé soluble ou dispersible dans une phase grasse liquide telle que définie précédemment, comprenant au moins un bloc styrène et au moins un bloc avec des motifs choisis parmi le butadiène, l'éthylène, le propylène, le butylène, l'isoprène ou un de leurs mélanges.

Le copolymère bloc hydrocarboné peut être notamment un copolymère dibloc, tribloc, multibloc, radial, étoile, ou leurs mélanges.
De tels copolymères blocs hydrocarbonés sont décrits dans la demande US-A-2002/005562 et dans le brevet US-A-5 221 534.

Le copolymère peut présenter au moins un bloc dont la température de transition vitreuse, est de préférence inférieure à 20°C, de préférence inférieure ou égale à 0°C, de préférence inférieure ou égale à -20°C, de préférence encore inférieure ou égale à -40°C. La température de transition vitreuse dudit bloc peut être comprise entre -150°C et 20°C, notamment entre -100°C et 0°C.

Dans ce cas, lorsque la résine est dotée d'au moins une température de transition vitreuse, l'écart entre les températures de transition vitreuse de la résine et du copolymère est généralement supérieur à 20°C, de préférence supérieur à 40°C, et mieux supérieur à 60°C.
Lorsque la résine est dotée d'au moins une température de transition vitreuse, le copolymère séquencé est avantageusement un plastifiant de la résine décrite précédemment. On entend par plastifiant de la résine, un composé, qui associé en quantité suffisante à la résine, abaisse la température de transition vitreuse de la résine telle que définie précédemment. Le composé plastifiant abaisse notamment la température de transition vitreuse du polymère d'au moins 2, 3 ou 4 °C, de préférence de 5°C à 20°C. Dans un mode de réalisation préféré, le composé plastifiant abaisse notamment la température de transition vitreuse du polymère d'au moins 2, 3 ou 4 °C, de préférence de 5°C à 20°C.

Le copolymère bloc hydrocarboné présent dans la composition selon l'invention est un copolymère amorphe.

Selon un mode préféré de réalisation, le copolymère bloc hydrocarboné est hydrogéné pour réduire les insaturations éthyléniques résiduelles après la polymérisation des monomères.

En particulier, le copolymère bloc hydrocarboné est un copolymère, éventuellement hydrogéné, à blocs styrène et à blocs éthylène/alkylène en C₃-C₄.

Comme copolymère dibloc, de préférence hydrogéné, on peut citer les copolymères de styrène-éthylène/propylène, les copolymères de styrène-éthylène/butadiène, les copolymères de styrène-éthylène/butylène. Des polymères diblocs sont notamment vendus sous la dénomination Kraton® G1701 E par la société Kraton Polymers.

Comme copolymère tribloc, de préférence hydrogéné, on peut citer les copolymères de styrène-éthylène/propylène-styrène, les copolymères de styrène-éthylène/butadiène-styrène, les copolymères de styrène-éthylène/butylène-styrène les copolymères de styrène-isoprène-styrène, les copolymères de styrène-butadiène-styrène. Des polymères triblocs sont notamment vendus sous les dénominations Kraton® G1650, Kraton® G1652, Kraton® D1101, Kraton® D1102, Kraton® D1160 par la société Kraton Polymers.

Selon un mode de réalisation de la présente invention, le copolymère bloc hydrocarboné est un copolymère tribloc styrène-éthylène/butylène-styrène.

Selon un mode préféré de réalisation de l'invention, on peut notamment utiliser un mélange d'un copolymère tribloc styrène-butylène/éthylène-styrène et d'un copolymère dibloc styrène-éthylène/butylène, notamment ceux vendus sous la dénomination Kraton® G1657M par la société Kraton Polymers.

Selon un mode préféré de réalisation, la résine est choisie parmi les copolymères indène/méthylstyrène/styrène hydrogéné.

De façon préférée, le rapport pondéral de la résine sur le copolymère bloc hydrocarboné allant de 1/1 à 4/1.

De façon encore préférée, le rapport pondéral de la résine sur le copolymère bloc hydrocarboné va de 1/1 à 3,5/1.

Le copolymère bloc hydrocarboné (ou le mélange de copolymères blocs hydrocarbonés) peut être présent en une teneur allant de 0,1 % à 15 % en poids, par rapport au poids total de la composition, de préférence allant de 0,5 % à 10 % en poids, et plus préférentiellement allant de 1 % à 8 % en poids.

### Phase grasse

La phase grasse de la composition selon l'invention comprend au moins un corps gras dont la température de fusion est supérieure à 25°C, choisi parmi les cires et les corps gras pâteux.

Au sens de l'invention, la température de fusion correspond à la température du pic le plus endothermique observé en analyse thermique (DSC) telle que décrite dans la norme ISO 11357-3 ; 1999. Le point de fusion d'un pâteux ou d'une cire peut être mesuré à l'aide d'un calorimètre à balayage différentiel (DSC), par exemple le calorimètre vendu sous la dénomination « MDSC 2920 » par la société TA Instruments.

### Le protocole de mesure est le suivant :

Un échantillon de 5 mg de pâteux ou de cire (selon le cas) disposé dans un creuset est soumis à une première montée en température allant de -20 °C à 100 °C, à la vitesse de chauffe de 10 °C/minute, puis est refroidi de 100 °C à -20 °C à une vitesse de refroidissement de 10 °C/minute et enfin soumis à une deuxième montée en température allant de -20 °C à 100 °C à une vitesse de chauffe de 5 °C/minute. Pendant la deuxième montée en température, on mesure la variation de la différence de puissance absorbée par le creuset vide et par le creuset contenant l'échantillon de cire en fonction de la température. Le point de fusion du composé est la valeur de la température correspondant au sommet du pic de la courbe représentant la variation de la différence de puissance absorbée en fonction de la température.

### Les pâteux

Par "pâteux" au sens de la présente invention, on entend un composé gras lipophile à changement d'état solide/liquide réversible, présentant à l'état solide une organisation cristalline anisotrope, et comportant à la température de 23°C une fraction liquide et une fraction solide.

En d'autres termes, la température de fusion commençante du composé pâteux peut être inférieure à 23°C. La fraction liquide du composé pâteux mesurée à 23°C peut représenter 9 à 97% en poids du composé. Cette fraction liquide à 23°C représente de préférence entre 15 et 85%, de préférence encore entre 40 et 85% en poids.

La fraction liquide en poids du composé pâteux à 23°C est égale au rapport de l'enthalpie de fusion consommée à 23°C sur l'enthalpie de fusion du composé pâteux.

L'enthalpie de fusion du composé pâteux est l'enthalpie consommée par le composé pour passer de l'état solide à l'état liquide. Le composé pâteux est dit à l'état solide lorsque l'intégralité de sa masse est sous forme solide cristalline. Le composé pâteux est dit à l'état liquide lorsque l'intégralité de sa masse est sous forme liquide.

L'enthalpie de fusion du composé pâteux est égale à l'aire sous la courbe du thermogramme obtenu à l'aide d'un calorimètre à balayage différentiel (D. S. C), tel que le calorimètre vendu sous la dénomination MDSC 2920 par la société TA instrument, avec une montée en température de 5 ou 10°C par minute, selon la norme ISO 11357-3:1999. L'enthalpie de fusion du composé pâteux est la quantité d'énergie nécessaire pour faire passer le composé de l'état solide à l'état liquide. Elle est exprimée en J/g.

L'enthalpie de fusion consommée à 23°C est la quantité d'énergie absorbée par l'échantillon pour passer de l'état solide à l'état qu'il présente à 23°C constitué d'une fraction liquide et d'une fraction solide.

La fraction liquide du composé pâteux mesurée à 32°C représente de préférence de 30 à 100% en poids du composé, de préférence de 50 à 100%, de préférence encore de 60 à 100% en poids du composé. Lorsque la fraction liquide du composé pâteux mesurée à 32°C est égale à 100%, la température de la fin de la plage de fusion du composé pâteux est inférieure ou égale à 32°C.

La fraction liquide du composé pâteux mesurée à 32°C est égale au rapport de l'enthalpie de fusion consommée à 32°C sur l'enthalpie de fusion du composé pâteux. L'enthalpie de fusion consommée à 32°C est calculée de la même façon que l'enthalpie de fusion consommée à 23°C.

Le composé pâteux au sens de l'invention, peut avoir une dureté à 20°C allant de 0,001 à 0,5 MPa, de préférence de 0,002 à 0,4 MPa.

La dureté est mesurée selon une méthode de pénétration d'une sonde dans un échantillon de composé et en particulier à l'aide d'un analyseur de texture (par exemple le TA-XT2i de chez Rhéo) équipé d'un cylindre en inox de 2 mm de diamètre. La mesure de dureté est effectuée à 20°C au centre de 5 échantillons. Le cylindre est introduit dans chaque échantillon à une pré-vitesse de 1 mm/s puis à une vitesse de mesure de 0,1 mm/s, la profondeur de pénétration étant de 0,3 mm. La valeur relevée de la dureté est celle du pic maximum.

### Le protocole de mesure est le suivant :

Le corps gras dont la température de fusion est supérieure à 25°C (pâteux ou cire selon le cas) est fondu à une température égale au point de fusion du corps gras + 10 °C. Le corps gras fondu est coulé dans un récipient de 25 mm de diamètre et de 20 mm de profondeur. Il est recristallisé à température ambiante (25 °C) pendant 24 heures de telle sorte que la surface du corps gras soit plane et lisse, puis le corps gras est conservé pendant au moins 1 heure à 20 °C avant d'effectuer la mesure de la dureté ou du collant.

Le mobile du texturomètre est déplacé à la vitesse de 0,1 mm/s, puis pénètre dans le corps gras jusqu'à une profondeur de pénétration de 0,3 mm. Lorsque le mobile a pénétré dans le corps gras à la profondeur de 0,3 mm, le mobile est maintenu fixe pendant 1 seconde (correspondant au temps de relaxation) puis est retiré à la vitesse de 0,5 mm/s.

La valeur de la dureté est la force de compression maximale mesurée divisée par la surface du cylindre du texturomètre en contact avec le corps gras.

Le composé pâteux est de préférence choisi parmi les composés synthétiques et les composés d'origine végétale. Un composé pâteux peut être obtenu par synthèse à partir de produits de départ d'origine végétale.

Le composé pâteux est avantageusement choisi parmi
- la lanoline et ses dérivés
- les éthers de polyol choisi parmi les éthers de pentaérythritol et de polyalkylène glycol, les éthers d'alcool gras et de sucre, et leurs mélanges. l'éther pentaérythritol et de polyéthylène glycol comportant 5 motifs oxyéthylénés (5 OE) (nom CTFA : PEG-5 Pentaerythrityl Ether), l'éther de pentaérythritol et de polypropylène glycol comportant 5 motifs oxypropylénés (5 OP) (nom CTFA : PPG-5 Pentaerythrityl Ether), et leurs mélanges et plus spécialement le mélange PEG-5 Pentaerythrityl Ether, PPG-5 Pentaerythrityl Ether et huile de soja, commercialisé sous la dénomination « Lanolide » par la société Vevy, mélange où les constituants se trouvent dans un rapport en poids 46/46/8 : 46 % de PEG-5 Pentaerythrityl Ether, 46 % de PPG-5 Pentaerythrityl Ether et 8 % d'huile de soja.
- les composés siliconés polymères ou non
- les composés fluorés polymères ou non
- les polymères vinyliques, notamment:
   - les homopolymères d'oléfines (tel que le polylaurate de vinyle)
   - les copolymères d'oléfines
   - les homopolymères et copolymères de diènes hydrogénés
   - les oligomères linéaires ou ramifiés, homo ou copolymères de (méth)acrylates d'alkyles ayant de préférence un groupement alkyle en C₈-C₃₀
   - les oligomères homo et copolymères d'esters vinyliques ayant des groupements alkyles en C₈-C₃₀
   - les oligomères homo et copolymères de vinyléthers ayant des groupements alkyles en C₈-C₃₀,
- les polyéthers liposolubles résultant de la polyéthérification entre un ou plusieurs diols en C2-C100, de préférence en C2-C50,
- les esters,
- et/ou leurs mélanges.

Le composé pâteux est de préférence polymère, notamment hydrocarboné.

Parmi les polyéthers liposolubles, on préfère en particulier les copolymères d'éthylène-oxyde et/ou de propylène-oxyde avec des alkylènes-oxydes à longue chaîne en C6-C30, de préférence encore tels que le rapport pondéral de l'éthylène-oxyde et/ou de propylène-oxyde avec alkylènes-oxydes dans le copolymère est de 5:95 à 70:30. Dans cette famille, on citera notamment les copolymères tels que les alkylènes-oxydes à longue chaîne sont disposés en blocs ayant un poids moléculaire moyen de 1.000 à 10.000, par exemple un copolymère bloc de polyoxyethylène/polydodécyle glycol tel que les éthers de dodécanediol (22 mol) et de polyéthylène glycol (45 OE) commercialisés sous la marque ELFACOS ST9 par Akzo Nobel.

Parmi les esters, on préfère notamment
- les esters d'un glycérol oligomère, notamment les esters de diglycérol, en particulier les condensats d'acide adipique et de glycérol, pour lesquels une partie des groupes hydroxyles des glycérols ont réagi avec un mélange d'acides gras tels que l'acide stéarique, l'acide caprique, l'acide stéarique et l'acide isostéarique et l'acide 12-hydroxystéarique, à l'image notamment de ceux commercialisé sous la marque Softisan 649 par la société Sasol
- le propionate d'arachidyle commercialisé sous la marque Waxenol 801 par Alzo,
- les esters de phytostérol,
- les triglycérides d'acides gras et leurs dérivés
- les esters de pentaérythritol
- les polyesters non réticulés résultant de la polycondensation entre un acide dicarboxylique
ou un polyacide carboxylique linéaire ou ramifié en C4-C50 et un diol ou un polyol en C2-C50,
- les esters aliphatiques d'ester résultant de l'estérification d'un ester d'acide hydroxycarboxylique aliphatique par un acide carboxylique aliphatique.

L'acide carboxylique aliphatique comprend de 4 à 30 et de préférence de 8 à 30 atomes de carbone. Il est de préférence choisi parmi l'acide héxanoïque, l'acide heptanoïque, l'acide octanoïque, l'acide éthyl-2 héxanoïque, l'acide nonanoïque, l'acide décanoïque, l'acide undécanoïque, acide dodécanoïque, l'acide tridécanoïque, l'acide tétradécanoïque, l'acide pentadécanoïque, l'acide héxadécanoïque, l'acide héxyldécanoïque, l'acide heptadécanoïque, l'acide octadécanoïque, l'acide isostéarique, l'acide nonadécanoïque, l'acide eicosanoïque, l'acide isoarachidique, l'acide octyldodécanoïque, l'acide henéicosanoïque, l'acide docosanoïque, et leurs mélanges.

L'acide carboxylique aliphatique est de préférence ramifié.
L'ester d'acide hydroxy carboxylique aliphatique est avantageusement issu d'un acide carboxylique aliphatique hydroxylé comportant de 2 à 40 atomes de carbone, de préférence de 10 à 34 atomes de carbone et mieux de 12 à 28 atomes de carbone, et de 1 à 20 groupes hydroxyle, de préférence de 1 à 10 groupes hydroxyle et mieux de 1 à 6 groupes hydroxyle. L'ester d'acide hydroxy carboxylique aliphatique est choisi parmi :
a) les esters partiels ou totaux d'acides monocarboxyliques aliphatiques mono hydroxylés linéaires, saturés ;
b) les esters partiels ou totaux d'acides monocarboxyliques aliphatiques mono hydroxylés insaturés ;
c) les esters partiels ou totaux de polyacides carboxyliques aliphatiques mono hydroxylés saturés ;
d) les esters partiels ou totaux de polyacides carboxyliques aliphatiques poly hydroxylés saturés ;
e) les esters partiels ou totaux de polyols aliphatiques en C₂ à C₁₆ ayant réagi avec un mono ou un poly acide carboxylique aliphatique mono ou poly hydroxylé,
   et leurs mélanges.

Les esters aliphatiques d'ester sont avantageusement choisis parmi :
- l'ester résultant de la réaction d'estérification de l'huile de ricin hydrogénée avec l'acide isostéarique dans les proportions 1 pour 1 (1/1) ou monoisostéarate d'huile de ricin hydrogénée,
- l'ester résultant de la réaction d'estérification de l'huile de ricin hydrogénée avec l'acide isostéarique dans les proportions 1 pour 2 (1/2) ou le diisostéarate d'huile de ricin hydrogénée,
- l'ester résultant de la réaction d'estérification de l'huile de ricin hydrogénée avec l'acide isostéarique dans les proportions 1 pour 3 (1/3) ou triisostéarate d'huile de ricin hydrogénée,
- et leurs mélanges.

Le composé pâteux représente de préférence 0,1 à 80%, mieux 0,5 à 60%, mieux 1 à 30% et mieux encore 1 à 15% en poids de la composition.

### Les cires

La cire considérée dans le cadre de la présente invention est d'une manière générale un composé lipophile, solide à température ambiante (25 °C), déformable ou non déformable, à changement d'état solide/liquide réversible, ayant un point de fusion supérieur ou égal à 30 °C pouvant aller jusqu'à 200 °C et notamment jusqu'à 120 °C.

En portant la cire à l'état liquide (fusion), il est possible de la rendre miscible aux huiles et de former un mélange homogène macroscopiquement, mais en ramenant la température du mélange à la température ambiante, on obtient une recristallisation de la cire dans les huiles du mélange.
En particulier, les cires convenant à l'invention peuvent présenter un point de fusion supérieur ou égal à 45 °C, et en particulier supérieur ou égal à 55°C.

A titre de cires pouvant être utilisées selon l'invention, on peut citer :
- les cires d'origine animale telles que la cire d'abeilles, le spermaceti, la cire de lanoline et les dérivés de lanoline, les cires végétales telles que la cire de Carnauba, de Candellila, d'Ouricury, du Japon, le beurre de cacao ou les cires de fibres de liège ou de canne à sucre,
- les cires minérales, par exemple de paraffine, de vaseline, de lignite ou les cires microcristallines ou les ozokérites,
- les cires synthétiques parmi lesquelles les cires de polyéthylène, et les cires obtenues par synthèse de Fisher-Tropsch,
- les cires de silicone, en particulier les polysiloxanes linéaires substitués; on peut citer, par exemple, les cires de silicone polyéther, les alkyl ou alkoxy-diméthicones ayant de 16 à 45 atomes de carbone, les alkyl méthicones comme la C₃₀-C₄₅ alkyl méthicone vendue sous la dénomination commerciale "AMS C 30" par DOW CORNING,
- les huiles hydrogénées concrètes à 25°C telles que l'huile de ricin hydrogénée, l'huile de jojoba hydrogénée, l'huile de palme hydrogénée, le suif hydrogéné, l'huile de coco hydrogénée et les esters gras concrets à 25°C comme le stéarate d'alkyle en C₂₀-C₄₀ vendu sous la dénomination commerciale "KESTER WAX K82H" par la société KOSTER KEUNEN,
- et/ou leurs mélanges.

De préférence, on utilisera les cires de polyéthylène, les cires microcristallines, les cires de carnauba, l'huile de jojoba hydrogénée, les cires de candellila, les cires d'abeilles et/ou leurs mélanges.

Les cires pouvant être utilisées dans les compositions selon l'invention présentent généralement une dureté allant de 0,01 MPa à 15 MPa, notamment supérieure à 0,05 MPa et en particulier supérieure à 0,1 MPa.

La dureté est déterminée par la mesure de la force en compression mesurée à 20 °C à l'aide du texturomètre vendu sous la dénomination TA-XT2 par la société RHEO, équipé d'un cylindre en inox d'un diamètre de 2 mm se déplaçant à la vitesse de mesure de 0,1 mm/s, et pénétrant dans la cire à une profondeur de pénétration de 0,3 mm.

Le protocole de mesure est le suivant :

La cire est fondue à une température égale au point de fusion de la cire + 10 °C. La cire fondue est coulée dans un récipient de 25 mm de diamètre et de 20 mm de profondeur. La cire est recristallisée à température ambiante (25 °C) pendant 24 heures de telle sorte que la surface de la cire soit plane et lisse, puis la cire est conservée pendant au moins 1 heure à 20 °C avant d'effectuer la mesure de la dureté ou du collant.

Le mobile du texturomètre est déplacé à la vitesse de 0,1 mm/s, puis pénètre dans la cire jusqu'à une profondeur de pénétration de 0,3 mm. Lorsque le mobile a pénétré dans la cire à la profondeur de 0,3 mm, le mobile est maintenu fixe pendant 1 seconde (correspondant au temps de relaxation) puis est retiré à la vitesse de 0,5 mm/s.

La valeur de la dureté est la force de compression maximale mesurée divisée par la surface du cylindre du texturomètre en contact avec la cire.

A titre illustratif des cires convenant à l'invention, on peut notamment citer les cires hydrocarbonées comme la cire d'abeilles, la cire de lanoline, et les cires d'insectes de Chine; la cire de son de riz, la cire de Carnauba, la cire de Candellila, la cire d'Ouricury, la cire d'Alfa, la cire de berry, la cire de shellac, la cire du Japon et la cire de sumac; la cire de montan, les cires d'orange et de citron, les cires microcristallines, les paraffines et l'ozokérite; les cires de polyéthylène, les cires obtenues par la synthèse de Fisher-Tropsch et les copolymères cireux ainsi que leurs esters.

On peut aussi citer des cires obtenues par hydrogénation catalytique d'huiles animales ou végétales ayant des chaînes grasses, linéaires ou ramifiées, en C₈-C₃₂- Parmi celles-ci, on peut notamment citer l'huile de jojoba isomérisée telle que l'huile de jojoba partiellement hydrogénée isomérisée trans fabriquée ou commercialisée par la société DESERT WHALE sous la référence commerciale Iso-Jojoba-50^{®}, l'huile de tournesol hydrogénée, l'huile de ricin hydrogénée, l'huile de coprah hydrogénée, l'huile de lanoline hydrogénée, et le tétrastéarate de di-(triméthylol-1,1,1 propane) vendu sous la dénomination de Hest 2T-4S^{®} par la société HETERENE.

On peut encore citer les cires de silicone, les cires fluorées.

On peut également utiliser les cires obtenues par hydrogénation d'huile de ricin estérifiée avec l'alcool cétylique vendues sous les dénominations de Phytowax ricin 16L64^{®} et 22L73^{®} par la société SOPHIM. De telles cires sont décrites dans la demande FR-A- 2792190.

On peut également utiliser en tant que cire un (hydroxystéaryloxy)stéarate d'alkyle en C₂₀-C₄₀ (le groupe alkyle comprenant de 20 à 40 atomes de carbone), seul ou en mélange.

Une telle cire est notamment vendue sous les dénominations « Kester Wax K 82 P^{®} » et « Kester Wax K 80 P^{®} » par la société KOSTER KEUNEN.

La composition selon l'invention peut comprendre une teneur en cires et/ou en corps gras pâteux dont la température de fusion est supérieure à 25°C allant de 0,1 à 20 % en poids par rapport au poids total de la composition, en particulier elle peut en contenir de 0,5 à 15 %, plus particulièrement de 1 à 12 %.

Ces corps gras peuvent en particulier être choisis de manière variée par l'homme du métier afin de préparer une composition ayant les propriétés souhaitées, par exemple en consistance ou en texture.

La phase grasse de la composition selon l'invention peut également comprendre des corps gras autres que les des cires ou encore des corps gras pâteux cires cités ci-dessus, tels que les huiles non volatiles.

La composition selon l'invention comprend en outre au moins une huile non volatile.

Par "huile non volatile", on entend une huile restant sur la peau à température ambiante et pression atmosphérique au moins plusieurs heures et ayant notamment une pression de vapeur inférieure à 0,13 Pa (0,01 mm de Hg).

Ces huiles non volatiles peuvent être des huiles hydrocarbonées notamment d'origine animale ou végétale, des huiles siliconées, ou leurs mélanges. On entend par "huile hydrocarbonée", une huile contenant principalement des atomes d'hydrogène et de carbone et éventuellement des atomes d'oxygène, d'azote, de soufre et/ou de phosphore.

Les huiles non volatiles peuvent notamment être choisies parmi les huiles hydrocarbonées le cas échéant fluorées et/ou les huiles siliconées non volatiles.

Comme huile hydrocarbonée non volatile, on peut notamment citer :
- les huiles hydrocarbonées d'origine animale,
- les huiles hydrocarbonées d'origine végétale telles que les triglycérides constitués d'esters d'acides gras et de glycérol dont les acides gras peuvent avoir des longueurs de chaînes variées de C₄ à C₂₄, ces dernières pouvant être linéaires ou ramifiées, saturées ou insaturées ; ces huiles sont notamment des triglycérides d'acide heptanoïque ou d'acide octanoïque, ou bien encore les huiles de germe de blé, de tournesol, de pépins de raisin, de sésame, de maïs, d'abricot, de ricin, de karité, d'avocat, d'olive, de soja, d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; le beurre de karité ; ou encore les triglycérides des acides caprylique/caprique comme ceux vendus par la société Stéarineries Dubois ou ceux vendus sous les dénominations Miglyol 810^{®}, 812^{®} et 818^{®} par la société Dynamit Nobel,
- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné tel que le Parleam ®, le squalane, les huiles de paraffine, et leurs mélanges,
- les esters de synthèse comme les huiles de formule R₁COOR₂ dans laquelle R₁ représente le reste d'un acide gras linéaire ou ramifié comportant de 1 à 40 atomes de carbone et R₂ représente une chaîne hydrocarbonée notamment ramifiée contenant de 1 à 40 atomes de carbone à condition que R₁ + R₂ soit ≥ 10, comme par exemple l'huile de Purcellin (octanoate de cétostéaryle), le myristate d'isopropyle, le palmitate d'isopropyle, les benzoates d'alcools en C₁₂ à C₁₅, le laurate d'hexyle, l'adipate de diisopropyle, l'isononanoate d'isononyle, le néopentanoate d'isodecyl, le palmitate de 2-éthyl-hexyle, l'isostéarate d'isostéaryle, le laurate de 2-hexyl-décyle, le palmitate de 2-octyl-décyle, le myristate de 2-octyl-dodécyle, des heptanoates, octanoates, décanoates ou ricinoléates d'alcools ou de polyalcools comme le dioctanoate de propylène glycol ; les esters hydroxylés comme le lactate d'isostéaryle, le malate de di-isostéaryle, le lactate de 2-octyl-dodécyle ; les esters de polyols et les esters du pentaérythritol,
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, et le 2-undécylpentadécanol,
- les acides gras supérieurs tels que l'acide oléique, l'acide linoléique, l'acide linolénique et leurs mélanges.

Les huiles de silicone non volatiles utilisables dans la composition selon l'invention peuvent être les polydiméthylsiloxanes (PDMS) non volatiles, les polydiméthylsiloxanes comportant des groupements alkyle ou alcoxy pendants et/ou en bouts de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, et leurs mélanges.

L'huile non volatile peut être présente dans la composition en une teneur allant de 10 à 90 % en poids, par rapport au poids total de la composition, de préférence allant de 10 à 80 % en poids.

La phase grasse peut être présente dans la composition selon l'invention en une teneur totale allant de 10 % à 95 % en poids, par rapport au poids total de la composition, de préférence allant de 15 % à 85 % en poids, de préférence allant de 20 % à 80 % en poids.

Comme indiqué précédemment, la composition selon l'invention est exempte d'huile volatile, c'est-à-dire qu'elle n'en comprend pas du tout, ou tout au moins qu'elle en comprend moins de 5 %, ou encore mieux, moins de 2 %, celle-ci n'étant alors présente qu'à l'état de « traces ».

Par « huile volatile », on entend au sens de l'invention toute huile susceptible de s'évaporer au contact de la peau, à température ambiante et pression atmosphérique. Les huiles volatiles de l'invention sont des huiles cosmétiques volatiles, liquides à température ambiante, ayant une pression de vapeur non nulle, à température ambiante et pression atmosphérique, allant en particulier de 0,13 Pa à 40.000 Pa (0,001 à 300 mm de Hg) et de préférence allant de 1,3 à 1300 Pa (0,01 à 10 mm de Hg).

### Agent épaississant des huiles additionnels:

La composition selon l'invention peut comprendre, en outre, au moins un agent épaississant des huiles choisi parmi les agents épaississants polymériques, les agents épaississants minéraux et leurs mélanges.

L'agent épaississant polymérique des huiles présent dans la composition selon l'invention peut être un polymère amorphe formé par polymérisation d'une oléfine. L'oléfine peut être notamment un monomère à insaturation éthylénique élastomérique.

Comme exemple d'oléfine, on peut citer les monomères de carbure éthylénique, ayant notamment une ou deux insaturations éthylénique, ayant de 2 à 5 atomes de carbone tels que l'éthylène, le propylène, le butadiène, l'isoprène.

L'agent épaississant polymérique des huiles est capable d'épaissir ou de gélifier la phase organique de la composition. Par polymère amorphe, on entend un polymère qui n'a pas de forme cristalline. L'agent épaississant polymérique peut être également filmogène, c'est-à-dire qu'il est capable de former un film lors de son application sur la peau.

L'agent épaississant polymérique des huiles peut notamment être choisi parmi:
- les polycondensats de type polyamide résultant de la condensation entre (α) au moins un acide choisi parmi les acides dicarboxyliques comprenant au moins 32 atomes de carbone tels que les acides gras dimères et (β) un alkylène diamine et en particulier l'éthylène diamine, dans lequel le polymère polyamide comprend au moins un groupe acide carboxylique terminal estérifié ou amidifié avec au moins un mono alcool ou une mono amine comprenant de 12 à 30 atomes de carbone linéaires et saturés, et en particulier, les copolymères d'éthylène diamine/dilinoléate de stéaryle tel que celui commercialisé sous la dénomination Uniclear 100 VG^{®} par la société ARIZONA CHEMICAL ;
- les polymères siliconés du type :
   1) des polyorganosiloxanes comportant au moins deux groupes capables d'établir des interactions hydrogène, ces deux groupes étant situés dans la chaîne du polymère, et/ou
   2) des polyorganosiloxanes comportant au moins deux groupes capables d'établir des interactions hydrogène, ces deux groupes étant situés sur des greffons ou ramifications. Les groupes capables d'établir des interactions hydrogène peuvent être choisis parmi les groupes ester, amide, sulfonamide, carbamate, thiocarbamate, urée, uréthane, thiourée, oxamido, guanidino, biguanidino et leurs combinaisons.

Les polymères siliconés utilisés comme agents structurants dans la composition de l'invention sont des polymères du type polyorganosiloxane comme par exemple ceux décrits dans les documents US-A-5 874 069, US-A-5,919,441, US-A-6,051,216 et US-A-5,981,680.

La composition selon l'invention peut également comprendre au moins un agent épaississant minéral des huiles tel qu'une argile organophile, les silices pyrogénées.

Les argiles organophiles sont des argiles modifiées par des composés chimiques rendant l'argile apte à gonfler dans les milieux huileux.

Les argiles sont des produits déjà bien connus en soi, qui sont décrits par exemple dans l'ouvrage "Minéralogie des argiles, S. Caillère, S. Hénin, M. Rautureau, 2ème édition 1982, Masson", dont l'enseignement est ici inclus à titre de référence. Les argiles sont des silicates contenant un cation pouvant être choisi parmi les cations de calcium, de magnésium, d'aluminium, de sodium, de potassium, de lithium et leurs mélanges.

A titre d'exemples de tels produits, on peut citer les argiles de la famille des smectites telles que les montmorillonites, les hectorites, les bentonites, les beidellites, les saponites, ainsi que de la famille des vermiculites, de la stévensite, des chlorites.

Ces argiles peuvent être d'origine naturelle ou synthétique. De préférence, on utilise les argiles qui sont cosmétiquement compatibles et acceptables avec les matières kératiniques comme la peau.

L'argile organophile peut être choisie parmi la montmorrilonite, la bentonite, l'hectorite, l'attapulgite, la sépiolite, et leurs mélanges. L'argile est de préférence une bentonite ou une hectorite.

Ces argiles peuvent être modifiées avec un composé chimique choisi parmi les amines quaternaires, les amines tertiaires, les acétates aminés, les imidazolines, les savons aminés, les sulfates gras, les alkyl aryl sulfonates, les oxides amines, et leurs mélanges.

Comme argiles organophiles, on peut citer les quaternium-18 bentonites telles que celles vendues sous les dénominations Bentone 3, Bentone 38, Bentone 38V par la société Rhéox, Tixogel VP par la société United catalyst, Claytone 34, Claytone 40, Claytone XL par la société Southern Clay; les stéaralkonium bentonites telles que celles vendues sous les dénominations Bentone 27 par la société Rheox, Tixogel LG par la société United Catalyst, Claytone AF, Claytone APA par la société Southern Clay ; les quaternium-18/benzalkonium bentonite telles que celles vendues sous les dénominations Claytone HT, Claytone PS par la société Southern Clay.

Les silices pyrogénées peuvent être obtenues par hydrolyse à haute température d'un composé volatil du silicium dans une flamme oxhydrique, produisant une silice finement divisée. Ce procédé permet notamment d'obtenir des silices hydrophiles qui présentent un nombre important de groupements silanol à leurs surface. De telles silices hydrophiles sont par exemple commercialisées sous les dénominations "AEROSIL 130^{®}", "AEROSIL 200^{®}", "AEROSIL 255^{®}", "AEROSIL 300^{®}", "AEROSIL 380^{®}" par la société Degussa, "CAB-O-SIL HS-5^{®}", "CAB-O-SIL EH-5^{®}", "CAB-O-SIL LM-130^{®}", "CAB-O-SIL MS-55^{®}", "CAB-O-SIL M-5^{®}" par la société Cabot.

Il est possible de modifier chimiquement la surface de ladite silice, par réaction chimique générant une diminution du nombre de groupes silanol. On peut notamment substituer des groupes silanol par des groupements hydrophobes : on obtient alors une silice hydrophobe.

Les groupements hydrophobes peuvent être :
- des groupements triméthylsiloxyl, qui sont notamment obtenus par traitement de silice pyrogénée en présence de l'hexaméthyldisilazane. Des silices ainsi traitées sont dénommées "Silica silylate" selon le CTFA (6ème édition, 1995). Elles sont par exemple commercialisées sous les références "AEROSIL R812^{®}" par la société Degussa, "CAB-O-SIL TS-530^{®}" par la société Cabot.
- des groupements diméthylsilyloxyl ou polydiméthylsiloxane, qui sont notamment obtenus par traitement de silice pyrogénée en présence de polydiméthylsiloxane ou du diméthyldichlorosilane. Des silices ainsi traitées sont dénomées "Silica diméthyl silylate" selon le CTFA (6^{ème} édition, 1995). Elles sont par exemple commercialisées sous les références "AEROSIL R972^{®}", "AEROSIL R974^{®}" par la société Degussa, "CAB-O-SIL TS-610^{®}", "CAB-O-SIL TS-720^{®}" par la société Cabot.

La silice pyrogénée présente de préférence une taille de particules pouvant être nanométrique à micrométrique, par exemple allant d'environ de 5 à 200 nm.

L'agent épaississant minéral des huiles peut être présent dans la composition selon l'invention en une teneur allant de 0,5 % à 7 % en poids, par rapport au poids total de la composition, de préférence en une teneur allant de 1 % à 5 % en poids, et préférentiellement allant de 1 % à 3 % en poids.

L'agent épaississant des huiles est présent dans la composition selon l'invention en une teneur totale allant de 0,1 à 20% en poids par rapport au poids total de la composition, de préférence allant de 0,5 à 15 % en poids, et plus préférentiellement allant de 1 à 10% en poids.

### Phase aqueuse

La composition selon l'invention peut comprendre une phase aqueuse.

La phase aqueuse comprend de l'eau. L'eau peut être une eau florale telle que l'eau de bleuet et/ou une eau minérale telle que l'eau de VITTEL, l'eau de LUCAS ou l'eau de LA ROCHE POSAY et/ou une eau thermale.

La phase aqueuse peut également comprendre des solvants organiques miscibles à l'eau (à température ambiante - 25 °C) comme par exemple :
- les polyols ayant notamment de 2 à 20 atomes de carbones, de préférence ayant de 2 à 10 atomes de carbone, et préférentiellement ayant de 2 à 6 atomes de carbone, tels que le glycérol, le propylène glycol, le butylène glycol, le pentylène glycol, l'hexylène glycol, le dipropylène glycol, le diéthylène glycol ;
- les éthers de glycol (ayant notamment de 3 à 16 atomes de carbone) tels que les alkyl(C₁-C₄)éther de mono, di- ou tripropylène glycol, les alkyl(C₁-C₄)éthers de mono, di- ou triéthylène glycol, et leurs mélanges.

La phase aqueuse peut comprendre en outre des agents de stabilisation, par exemple le chlorure de sodium, le dichlorure de magnésium et le sulfate de magnésium.

La phase aqueuse peut également comprendre tout composé hydrosoluble ou hydrodispersible compatible avec une phase aqueuse tels que des gélifiants, des polymères filmogènes, des épaississants, des tensioactifs et leurs mélanges.

De préférence, la phase aqueuse peut être présente dans la composition selon l'invention en une teneur allant de 1 à 95 % en poids, par rapport au poids total de la composition, et de préférence de 3 à 80 % en poids, et plus préférentiellement de 5 à 60 % en poids.

### Phase pulvérulente

La composition selon l'invention peut comprendre une phase pulvérulente notamment choisie parmi les pigments, les charges et/ou les nacres et leurs mélanges.

Selon un mode préféré de réalisation, la composition selon l'invention peut comprendre des pigments.

Par « pigments », il faut comprendre des particules, minérales ou organiques, insolubles dans la phase organique liquide, destinées à colorer et/ou opacifier la composition.

Les pigments peuvent être des pigments minéraux ou organiques. Comme pigments, on peut utiliser les oxydes métalliques comme les oxydes de fer (notamment ceux de couleur jaune, rouge, brun, noir), les dioxydes de titane, l'oxyde de cérium, l'oxyde de zirconium, l'oxyde de chrome ; le violet de manganèse, l'ultramarine bleue, le bleu de prusse, le bleu outremer, le bleu ferrique, l'oxychlorure de bismuth, la nacre, le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth, et leurs mélanges.
On utilise de préférence des pigments d'oxydes de fer ou de dioxyde de titane.

Les pigments peuvent être traités avec un agent hydrophobe pour les rendre compatible avec la phase organique de la composition. L'agent de traitement hydrophobe peut être choisi parmi les silicones comme les méthicones, les diméthicones, les perfluoroalkylsilanes ; les acides gras comme l'acide stéarique ; les savons métalliques comme le dimyristate d'aluminium, le sel d'aluminium du glutamate de suif hydrogéné, les perfluoroalkyl phosphates, les perfluoroalkyl si lanes, les perfluoroalkyl silazanes, les polyoxydes d'hexafluoropropylène, les polyorganosiloxanes comprenant des groupes perfluoroalkylles perfluoropolyéthers, les acides aminés ; les acides aminés N-acylés ou leurs sels ; la lécithine, le trisostéaryle titanate d'isopropyle, et leurs mélanges.
Les acides aminés N-acylés peuvent comprendre un groupe acyle ayant de 8 à 22 atomes de carbones, comme par exemple un groupe 2-éthyl hexanoyle, caproyle, lauroyle, myristoyle, palmitoyle, stéaroyle, cocoyle. Les sels de ces composés peuvent être les sels d'aluminium, de magnésium, de calcium, de zirconium, de zin, de sodium, de potassium. L'acide aminé peut être par exemple la lysine, l'acide glutamique, l'alanine

Le terme alkyl mentionné dans les composés cités précédemment désigne notamment un groupe alkyle ayant de 1 à 30 atomes de carbone, de préférence ayant de 5 à 16 atomes de carbone.
Des pigments traités hydrophobes sont notamment décrits dans la demande EP-A-1 086683.

Les pigments peuvent être présents, dans la composition selon l'invention, en une teneur supérieure ou égale à 0,01 à 50% en poids, par rapport au poids total de la composition, en particulier allant de 0,1% à 30% en poids, préférentiellement allant de 0,5% à 20% en poids, en particulier allant de 0,5 % à 15% en poids.

Outre les pigments, la phase pulvérulente de la composition selon l'invention peut comprendre des charges et/ou des nacres.

Selon un mode préféré de réalisation, la composition selon l'invention peut comprendre des charges.

Par charges, il faut comprendre des particules de toute forme, incolores ou blanches, minérales ou de synthèse, insolubles dans le milieu de la composition quelle que soit la température à laquelle la composition est fabriquée.

Les charges peuvent être minérales ou organiques de toute forme, plaquettaires, sphériques ou oblongues, quelle que soit la forme cristallographique (par exemple feuillet, cubique, hexagonale, orthorombique, etc) . On peut citer le talc, le mica, la silice, le kaolin, les poudres de polyamide (Nylon®) , les poudres de poly-β-alanine, les poudres de polyéthylène, les polyméthacrylates de métyle, les poudres de polyuréthane telle que la poudre de copolymère de diisocyanate d'hexaméthylène et de triméthylol hexyl lactone vendue sous les dénominations PLASTIC POWDER D-400 par la société TOSHIKI, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique, les poudres de résine de silicone, en particulier les poudres de silsesquioxane (poudres de résine de silicone notamment décrites dans le brevet EP 293795 ; Tospearls® de Toshiba, par exemple), les particules de polyorganosiloxanes élastomères, le carbonate de calcium précipité, le carbonate et l'hydro-carbonate de magnésium, l'hydroxyapatite, les microsphères de silice creuses, les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium ; le sulfate de baryum et leurs mélanges.

Les charges peuvent être présentes dans la composition selon l'invention en une teneur totale allant de 0,01 % à 99 % en poids, par rapport au poids total de la composition, de préférence allant de 0,02 % à9 % en poids, et préférentiellement allant de 0,05 % à 90 % en poids.

Outre les pigments et les charges, la phase particulaire de la composition selon l'invention peut comprendre des nacres.

Par « nacres », il faut comprendre des particules irisées, notamment produites par certains mollusques dans leur coquille ou bien synthétisées, insolubles dans le milieu de la composition.

Les nacres peuvent être choisies parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les nacres peuvent être présents la composition selon l'invention en une teneur allant de 0,01 % à 50 % en poids, par rapport au poids total de la composition, de préférence allant de 0,02 % à 30 % en poids, et préférentiellement allant de 0,5 % à 20 % en poids.

### Colorants additionnels

La composition selon l'invention peut comprendre des colorants additionnels choisis parmi les colorants hydrosolubles et liposolubles.

Les colorants hydrosolubles sont par exemple le jus de betterave, le bleu de méthylène, le caramel.

Par colorants liposolubles, il faut comprendre des composés généralement organiques solubles dans les corps gras comme les huiles. Les colorants liposolubles sont par exemple le rouge Soudan, le D&C Red n° 17, le D&C Green n° 6, le β-carotène, l'huile de soja, le brun Soudan, le D&C Yellow n° 11, le D&C Violet n° 2, le D&C orange n°5, le jaune quinoléine, le rocou, les bromoacides.

Les colorants additionnels peuvent être présents la composition selon l'invention en une teneur allant de 0,001 % à 30 % en poids, par rapport au poids total de la composition, de préférence allant de 0,01% à 20 % en poids, et préférentiellement allant de 0,02 % à 10 % en poids.

### Ingrédients cosmétiques usuels additionnels

La composition selon l'invention peut comprendre au moins un autre ingrédient cosmétique usuel pouvant être choisi notamment parmi les antioxydants, les parfums, les conservateurs, les neutralisants, les tensioactifs, les filtres solaires, les vitamines, les hydratants, les composés auto-bronzants, les actifs antirides, les émollients, les actifs hydrophiles ou lipophiles, les agents anti-radicaux libres, les agents déodorants, les sequestrants, les agents filmogènes, et leurs mélanges.

L'invention a également pour objet, selon un autre aspect, un procédé de maquillage et/ou de soin de la peau consistant à appliquer sur la peau une composition comprenant une phase grasse liquide comprenant une résine de poids moléculaire moyen en nombre inférieur ou égal à 10000 g/mol, choisie parmi les résines hydrocarbonées et leurs mélanges, et au moins un copolymère bloc hydrocarboné choisi parmi les copolymères dibloc de styrène-éthylène/propylène, de styrène-éthylène/butadiène, et les copolymères tribloc de styrène-éthylène/butadiène-styrène, de styrène-isoprène-styrène et de styrène-butadiène-styrène, la composition comprenant en outre au moins un corps gras dont la température de fusion est supérieure à 25°C, choisi parmi les cires ou les corps gras pâteux, la composition ne comprenant pas d'huile volatile ou en contenant moins de 5% ou mieux, moins de 2%, la composition comprenant en outre au moins une huile non volatile et étant sous forme liquide à température ambiante (252°C).

Selon un mode préféré de réalisation, la résine de la composition du procédé de maquillage et/ou de soin est choisie parmi les copolymères indène/méthylstyrène/styrène hydrogéné.

L'invention est présentée plus en détail dans les exemples illustratifs mais non limitatifs ci-après.

### Exemples 1 à 4 :

On a préparé des rouges à lèvres liquides selon l'invention ayant la formule générale suivante :

| Etape | COMPOSITION | Type | Concentration en % massique |
|---|---|---|---|
| Prégel | ISOSTEARATE D'ISOPROPYLE | CORPS GRAS | 1,3 |
| Prégel | STYRENE-ETHYLENE/BUTYLENE-STYRENE BLOCK COPOLYMER (KRATON®) | POLYMERE | 7,5 |
| Prégel | POLYDECENE HYDROGENE (VISCOSITE: 54 CPS) | POLYMERE | 10 |
| Prégel | POLYISOBUTENE HYDROGENE | POLYMERE | 18,5 |
| Prégel | COPOLYMERE STYRENE / METHYL STYRENE / INDENE HYDROGENE (REGALITE® R 1100) | POLYMERE | 15 |
| Prégel (moitié en masse) | NEOPENTANOATE D'OCTYL-2 DODECYLE (QS) | CORPS GRAS | 26,029 |
| Prégel | CORPS GRAS DONT LA TEMPERATURE DE FUSION EST SUPERIEURE A 25°C | PATEUX OU CIRE | 5 |
| | N-LAUROYL L-LYSINE | CHARGE | 1 |
| | SEL DE CALCIUM DU ROUGE LITHOL B | COLORANT | 0,225 |
| | OXYDES DE FER BRUN,JAUNE (75/25) (CI: 77491 + 77492) | COLORANT | 0,255 |
| | POLYBUTENE (MONOOLEFINES / ISOPARAFFINES 95/5)(PM : 2060) | POLYMERE | 10 |
| | SILICE PYROGENEE HYDROPHOBE,TRAITEE EN SURFACE PAR DI-METHYLSILANE | CHARGE | 5 |
| | OXYDE DE FER NOIR (CI: 77499) | COLORANT | 0,041 |
| | OXYDE DE TITANE RUTILE TRAITE ALUMINE/SILICE/TRI-METHYOLPROPANE (CI: 77891) | COLORANT | 0,15 |
| | TOTAL : | | 100 |

### Mode opératoire :

Préparation du prégel :
Dans un poëlon à double paroi, on mélange le polymère bloc Kraton® avec le polyisobutène hydrogéné, l'isostéarate d'isopropyle et la moitié en masse du néopentanoate d'octyl-2 dodécyle à l'aide d'un Rayneri.

Ce mélange est chauffé à 100°C pendant deux heures pour obtenir un gel transparent et homogène à 100°C. Puis, toujours à 100°C, on ajoute la Régalite. Le mélange est laissé sous agitation pendant une heure à 100°C.

On ajoute ensuite le corps gras dont la température de fusion est supérieure à 25°C, le mélange étant toujours maintenu à 100°C.

Les différents corps gras dont la température de fusion est supérieure à 25°C utilisés pour la réalisation des différents prégels sont :
Exemple 1 : l'huile de ricin hydrogénée dimère dilinoléate (RISOCAST DA-L commercialisé par Kokyu Alcohol Kogyo) qui est un corps gras pâteux.
Exemple 2 : l'huile de ricin hydrogénée isostéarate (SALACOS HCIS(V)-L commercialisé par Nisshin Oil) qui est un corps gras pâteux.
Exemple 3 : le bis-diglyceryl polyacyladipate-2 (SOFTISAN 645 commercialisé par Sasol) qui est un corps gras pâteux.
Exemple 4 : le PEG-45/dodécyl glycol copolymère (ELFACOST ST9 commercialisé par Azko Nobel) qui est un corps gras pâteux.

Fin de la préparation de la composition :

Les colorants et les charges sont broyés dans l'autre moitié en masse du néopentanoate d'octyl-2 dodécyle. Ce broyat est ajouté toujours à 100°C dans le prégel préparé précédemment. On ajoute ensuite le polybutène, puis la silice pyrogénée hydrophobe que l'on disperse au Rayneri toujours à une température de 100°C.

Toute la phase de préparation se fait ainsi à 100°C. On obtient après refroidissement à température ambiante un rouge à lèvres liquide dont le dépôt est brillant et dont la brillance est rémanente. De plus, on observe un faible niveau de migration.

### Exemple 5 :

On a préparé un rouge à lèvre liquide selon l'invention ayant la formule générale suivante :

| Etape | COMPOSITION | Type | Concentration en % massique |
|---|---|---|---|
| | N-LAUROYL L-LYSINE | CHARGE | 1 |
| Prégel | ESTERS D'ACIDES GRAS VEGETAUX,ISO-STEARIQUE,ADIPIQUE DE GLYCERYLE | CORPS GRAS PATEUX | 9 |
| Prégel | ISOSTEARATE D'ISOPROPYLE | CORPS GRAS | 2,3 |
| | SEL DE CALCIUM DU ROUGE LITHOL B | COLORANT | 0,225 |
| | OXYDES DE FER BRUN,JAUNE (75/25) (CI: 77491 + 77492) | COLORANT | 0,255 |
| Prégel | HYDROGENATED POLYISOBUTENE | POLYMERE | 23,5 |
| Prégel | COPOLYMERE STYRENE / METHYL STYRENE / INDENE HYDROGENE (REGALITE® R 1100) | POLYMERE | 15 |
| | SILICE PYROGENEE HYDROPHOBE,TRAITEE EN SURFACE PAR DI-METHYLSILANE | CHARGE | 5 |
| | POLYBUTENE (MONOOLEFINES / ISOPARAFFINES 95/5)(PM : 2060) | POLYMERE | 12 |
| Prégel | STYRENE-ETHYLENE/BUTYLENE-STYRENE BLOCK COPOLYMER (KRATON®) | POLYMERE | 7,5 |
| | OXYDE DE FER NOIR (CI: 77499) | COLORANT | 0,041 |
| | OXYDE DE TITANE RUTILE TRAITE ALUMINE/SILICE/TRI-METHYOLPROPANE (CI: 77891) | COLORANT | 0,15 |
| Prégel (moitié en masse) | NEOPENTANOATE D'OCTYL-2 DODECYLE (QS) | CORPS GRAS | 24,029 |
| | Total : | | 100 |

### Mode opératoire

Le mode opératoire utilisé est le même que celui décrit précédemment.

### Résultats

Le rouge à lèvres a été appliqué sur un panel de six femmes. On observe un très bon niveau de brillance ainsi que rémanence de la brillance pendant au moins 4 heures après application.

Les exemples 1 à 5 montrent donc que l'association de la résine copolymère styrène/méthyl styrène/indène hydrogéné Régalite R 1100, du Kraton et d'un pâteux en l'absence de volatil permet d'obtenir un dépôt ayant un bon niveau de brillance et une très bonne rémanence de la brillance après application pendant au moins 4 heures.

### Exemple 6 : comparatif

On a préparé un rouge à lèvres liquide ayant la composition suivante et ne contenant pas de corps gras dont la température de fusion est supérieure à 25°C :

| Etape | COMPOSITION | Type | Concentration en % massique |
|---|---|---|---|
| | N-LAUROYL L-LYSINE | CHARGE | 1 |
| Prégel | ISOSTEARATE D'ISOPROPYLE | CORPS GRAS | 1,35 |
| | OXYDE DE TITANE RUTILE TRAITE ALUMINE/SILICE/TRI-METHYOLPROPANE (Cl: 77891) | COLORANT | 0,15 |
| | OXYDE DE FER NOIR (CI: 77499) | COLORANT | 0,041 |
| Prégel | STYRENE-ETHYLENE/BUTYLENE-STYRENE BLOCK COPOLYMER (KRATON®) | POLYMERE | 7,4 |
| | POLYBUTENE (MONOOLEFINES / ISOPARAFFINES)(PM : 920) | POLYMERE | 15 |
| | SILICE PYROGENEE HYDROPHOBE,TRAITEE EN SURFACE PAR DI-METHYLSILANE | CHARGE | 5 |
| Prégel | COPOLYMERE STYRENE / METHYL STYRENE / INDENE HYDROGENE (REGALITE® R 1100) | POLYMERE | 22,1 |
| Prégel | POLYDECENE HYDROGENE (PM: 549 - 34% TRIMERE, 44% TETRAMERE, 17% PENTAMERE, 4% HEXAMERE) | POLYMERE | 14 |
| | OXYDES DE FER BRUN,JAUNE (75/25) (CI: 77491 + 77492) | COLORANT | 0,255 |
| | SEL DE CALCIUM DU ROUGE LITHOL B | COLORANT | 0,225 |
| Prégel (moitié en masse) | NEOPENTANOATE D'OCTYL-2 DODECYLE (QS) | CORPS GRAS | 33,479 |
| | Total : | | 100 |

Le mode de préparation de la composition est le même que dans les exemples précédents.

Ce rouge à lèvres est comme dans le cas de l'exemple 5 appliqué sur un panel de six femmes. On observe la brillance du dépôt et la rémanence de la brillance pendant 4 heures après l'application.

La comparaison des rouges à lèvres des exemples 5 (invention) et 6 (comparatif) permet de montrer que l'on obtient une meilleure rémanence de la brillance dans le cas de la composition de l'exemple 5 comprenant un corps gras pâteux pour lequel on n'observe pas de baisse du niveau de brillance pendant au moins 4 heures.

De plus, la composition selon l'invention de l'exemple 5 migre moins que la composition de l'exemple comparatif 6.

### Exemple 7 :

On a préparé un rouge à lèvres solide selon l'invention ayant la composition suivante :

| | COMPOSITION | Type | Concentration en % massique |
|---|---|---|---|
| Prégel (moitié en masse) | NEOPENTANOATE D'OCTYL-2 DODECYLE | CORPS GRAS | 20,6 |
| Prégel | ESTERS D'ACIDES GRAS VEGETAUX,ISO-STEARIQUE,ADIPIQUE DE GLYCERYLE | CORPS GRAS PATEUX | 7,1 |
| Prégel | ISOSTEARATE D'ISOPROPYLE | CORPS GRAS | 6,55 |
| | OXYDE DE TITANE RUTILE TRAITE ALUMINE/SILICE/TRI-METHYOLPROPANE (CI: 77891) | COLORANT | 0,2 |
| | SEL DE CALCIUM DU ROUGE LITHOL B | COLORANT | 0,45 |
| | LAQUE D'ALUMINIUM DE BLEU BRILLANT FCF SUR ALUMINE (12/88) (CI: 42090:2 + 77002) | COLORANT | 0,2 |
| | OXYDES DE FER BRUN,JAUNE (75/25) (Cl: 77491 + 77492) | COLORANT | 0,95 |
| Prégel | HOMOPOLYMERE DE L'ETHYLENE | CIRE | 4,92 |
| Prégel | COPOLYMERE STYRENE / METHYL STYRENE / INDENE HYDROGENE (REGALITE® R 1100) | POLYMERE | 15 |
| Prégel | MÉLANGE D'ALCOOL GRAS A LONGUE CHAINE LINEAIRE (C30-C50) ET D'HYDROCARBURE À MÊME NOMBRE DE CARBONE (80/20) | CIRE | 0,5 |
| Prégel | CIRE DE POLYETHYLENE (PM: 500) | CIRE | 5,88 |
| | LAQUE D'ALUMINIUM DE TARTRAZINE SUR ALUMINE (15/85) (CI: 19140:1 + 77002) | COLORANT | 0,85 |
| | MICA-OXYDE DE TITANE (59/41) (CI: 77019 + 77891) | NACRE | 2,8 |
| | MICA-OXYDE DE TITANE (52/48) (CI: 77019 + 77891) | NACRE | 1 |
| | MICA-OXYDE DE TITANE (60/40) (CI: 77019 + 77891) | NACRE | 0,5 |
| Prégel | STYRENE-ETHYLENE/BUTYLENE-STYRENE BLOCK COPOLYMER (KRATON®) | POLYMERE | 7,5 |
| Prégel (moitié en masse) | HYDROGENATED POLYISOBUTENE (QS) | POLYMERE | 25 |
| | Total : | | 100 |

### Mode opératoire

Préparation du prégel :
Dans un poëlon à double paroi, on mélange le polymère bloc Kraton® avec la moitié en masse du polyisobutène hydrogéné, l'isostéarate d'isopropyle et la moitié en masse du néopentanoate d'octyl-2 dodécyle à l'aide d'un Rayneri.
Ce mélange est chauffé à 100°C pendant deux heures pour obtenir un gel transparent et homogène à 100°C. Puis, toujours à 100°C, on ajoute la Régalite. Le mélange est laissé sous agitation pendant une heure à 100°C.
On ajoute ensuite les corps gras dont la température de fusion est supérieure à 25°C, à savoir le corps gras pâteux et les trois différentes cires, le mélange étant toujours maintenu à 100°C.

Fin de la préparation de la composition :

Les colorants sont broyés dans l'autre moitié en masse du néopentanoate d'octyl-2 dodécyle et du polyisobutène hydrogéné. Ce broyat est ajouté toujours à 100°C dans le prégel préparé précédemment. On ajoute ensuite les nacres toujours à une température de 100°C.

Toute la phase de préparation se fait ainsi à 100°C. Le rouge à lèvres est ensuite coulé dans un moule et laissé refroidir pendant 24 heures à 20°C. On prépare ainsi un stick de ladite composition ayant une section circulaire de 12,7 mm de diamètre.

Le stick ainsi obtenu permet l'obtention d'un dépôt homogène, brillant et dont la brillance est rémanente. De plus, on observe un faible niveau de migration.

## Revendications

1. Composition de maquillage ou de soin de la peau comprenant une phase grasse comprenant:
- au moins une résine de poids moléculaire moyen en nombre inférieur ou égal à 10 000 g/mol, choisie parmi les résines hydrocarbonées et leurs mélanges,
- au moins un copolymère bloc hydrocarboné choisi parmi les copolymères séquencés, éventuellement hydrogénés, comprenant au moins un bloc styrène et au moins un bloc comprenant des motifs choisis parmi le butadiène, l'éthylène, le propylène, le butylène, l'isoprène ou un de leurs mélanges,
- au moins un corps gras dont la température de fusion est supérieure à 25°C choisi parmi les cires et les corps gras pâteux,
ladite composition ne comprenant pas d'huile volatile, ou contenant moins de 5% en poids, ou mieux moins de 2% en poids d'huile volatile par rapport au poids total de la composition,
- au moins une huile non volatile,
et ladite composition étant sous forme liquide à température ambiante (25°C).

2. Composition selon la revendication 1, **caractérisée en ce qu'**elle est anhydre, ou **en ce qu'**elle comprend moins de 10% d'eau en poids ou mieux moins de 4%, par rapport au poids total de la composition.

3. Composition selon l'une quelconque des revendications précédentes, **caractérisée en ce qu'**elle ne comprend pas de composé siliconé, ou **en ce qu'**elle comprend moins de 10% en poids de composé siliconé ou mieux moins de 4%, par rapport au poids total de la composition.

4. Composition selon l'une quelconque des revendications précédentes, **caractérisée en ce que** la résine présente un poids moléculaire moyen en nombre allant de 250 à 10000 g/mol.

5. Composition selon la revendication précédente, **caractérisée en ce que** la résine présente un poids moléculaire moyen en nombre inférieur ou égal 5000 g/mol, notamment allant de 250 à 5000 g/mol, de préférence inférieur ou égal à 2000 notamment allant de 250 à 2000 g/mol et / préférentiellement inférieur ou égal à 1000 g/mol, notamment allant de 250 à 1000 g/mol.

6. Composition selon l'une quelconque des revendications précédentes, **caractérisée en ce que** les résines hydrocarbonées sont choisies parmi les résines hydrocarbonées indéniques, les résines aliphatiques de pentanediène, les résines mixtes de pentanediène et d'indène, les résines diènes des dimères de cyclopentanediène, les résines diènes des dimères de l'isoprène et leurs mélanges.

7. Composition selon l'une quelconque des revendications précédentes, **caractérisée en ce que** la résine est une résine hydrocarbonée indénique issue de la polymérisation de monomère indène et de monomère choisi parmi le styrène, le méthylindène, le méthylstyrène et leurs mélanges.

8. Composition selon la revendication précédente, **caractérisée en ce que** la résine hydrocarbonée indénique est hydrogénée.

9. Composition selon l'une quelconque des revendications 1 à 8, **caractérisée en ce que** la résine est une résine indénique choisie parmi les copolymères indène/méthylstyrène/styrène hydrogénés.

10. Composition selon l'une quelconque des revendications 1 à 8, **caractérisée en ce que** la résine est une résine aliphatique de pentanediène issue de la polymérisation du monomère 1,3-pentanediène (trans ou cis pipérylène) et de monomère choisi parmi l'isoprène, le butène le 2-méthyl-2-butène, le pentène, le 1, 4-pentanediène et leurs mélanges.

11. Composition selon l'une quelconque des revendications 1 à 8, **caractérisée en ce que** la résine est une résine mixte de pentanediène et d'indène.

12. Composition selon l'une quelconque des revendications 1 à 8, **caractérisée en ce que** la résine est une résine diène des dimères de cyclopentanediène issue de la polymérisation d'un premier monomère choisi parmi l'indène et le styrène, et d'un deuxième monomère choisi parmi les dimères du cyclopentanediène.

13. Composition selon l'une quelconque des revendications 1 à 8, **caractérisée en ce que** la résine est une résine diène des dimères de l'isoprène issue de la polymérisation d'au moins un monomère choisi parmi l'α-pinène, le β-pinène, le limonène, et leurs mélanges.

14. Composition selon l'une quelconque des revendications précédentes, **caractérisée en ce que** la résine est présente en une teneur allant de 0,1 à 30 % en poids, par rapport au poids total de la composition, de préférence allant de 0,3 à 20 % en poids, plus préférentiellement allant de 0,5 à 15 % en poids.

15. Composition selon l'une quelconque des revendications précédentes, **caractérisée en ce que** ladite cire est choisie parmi :
- les cires hydrocarbonées,
- les cires obtenues par hydrogénation catalytique d'huiles animales ou végétales ayant des chaînes grasses, linéaires ou ramifiées, en C₈-C₃₂,
- les huiles hydrogénées concrètes à 25°C,
- les cires de silicone,
- les cires fluorées,
- et/ou leurs mélanges.

16. Composition selon l'une quelconque des revendications précédentes, caractérisée en ce ledit corps gras pâteux est choisi parmi :
- la lanoline et ses dérivés
- les éthers de polyol choisi parmi les éthers de pentaérythritol et de polyalkylène glycol, les éthers d'alcool gras et de sucre, et leurs mélanges.
- les composés siliconés polymères ou non
- les composés fluorés polymères ou non
- les polymères vinyliques, notamment:
- les polyéthers liposolubles résultant de la polyéthérification entre un ou plusieurs diols en C2-C100, de préférence en C2-C50,
- les esters,
- et/ou leurs mélanges.

17. Composition selon l'une quelconque des revendications précédentes, caractérisée en ce le ou lesdits corps gras solides à 25°C sont présents dans la composition en une teneur allant de 0,1 à 20 % en poids, par rapport au poids total de la composition, de préférence allant de 0,5 à 15 % en poids.

18. Composition selon la revendication précédente, **caractérisée en ce que** la ladite huile non volatile est présente dans la composition en une teneur totale allant de 10 % à 90 % en poids, par rapport au poids total de la composition, de préférence allant de 10 % à 80 % en poids

19. Composition selon l'une quelconque des revendications précédentes, **caractérisée en ce que** la phase grasse de la composition est présente dans la composition en une teneur totale allant de 10 % à 95 % en poids, par rapport au poids total de la composition, de préférence allant de 15 % à 85 % en poids.

20. Composition selon l'une des revendications précédentes, **caractérisée par le fait que** le copolymère bloc hydrocarboné est un copolymère, éventuellement hydrogéné, à blocs styrène et à blocs éthylène/alkylène en C₃-C₄.

21. Composition selon la revendication précédente, **caractérisée par le fait que** le copolymère bloc hydrocarboné est choisi parmi les copolymères dibloc, éventuellement hydrogénés, de styrène-éthylène/propylène, de styrène-éthylène/butadiène, de styrène-étylène/butylène et les copolymères tribloc, éventuellement hydrogénés, de styrène-éthylène/butadiène-styrène, de styrène-butylène/éthylène-styrène de styrène-isoprène-styrène et de styrène-butadiène-styrène.

22. Composition selon l'une quelconque des revendications 20 et 21, **caractérisée par le fait que** le copolymère bloc hydrocarboné est un mélange de copolymère hydrogéné tribloc de styrène-butylène/éthylène-styrène et de copolymère dibloc de styrène-étylène/butylène.

23. Composition selon l'une quelconque des revendications 20 à 22, **caractérisée en ce que** le copolymère bloc hydrocarboné est présent dans la composition en une teneur allant de 0,1 % à 15 % en poids, par rapport au poids total de la composition, de préférence allant de 0,5 % à 10 % en poids, et plus préférentiellement allant de 1 % à 8 % en poids.

24. Composition selon l'une quelconque des revendications précédentes, **caractérisé en ce que** le fait que le rapport pondéral de la résine sur le copolymère bloc hydrocarboné va de 1/1 à 4/1.

25. Composition selon l'une quelconque des revendications précédentes, **caractérisé en ce que** la composition comprend au moins une phase pulvérulente choisie parmi les pigments, les nacres, et leurs mélanges.

26. Composition selon l'une quelconque des revendications précédentes, **caractérisé en ce que** la composition comprend une phase aqueuse en une teneur allant de 3% à 80% en poids, par rapport au poids total de la composition, de préférence allant de 5 à 60% en poids.

27. Composition selon l'une quelconque des revendications précédentes, **caractérisé en ce que** la composition comprend un ingrédient cosmétique choisi parmi les antioxydants, les parfums, les conservateurs, les neutralisants, les tensioactifs, les filtres solaires, les vitamines, les hydratants, les composés auto-bronzants, les actifs antirides, les émollients, les actifs hydrophiles ou lipophiles, les agents anti-radicaux libres, les sequestrants, les agents déodorants, les agents filmogènes, et leurs mélanges.

28. Procédé non thérapeutique de maquillage ou de soin de la peau comprenant l'application sur la peau d'une composition selon l'une quelconque des revendications 1 à 27.

29. Utilisation d'une composition selon l'une quelconque des revendications 1 à 27, pour obtenir un dépôt, notamment un maquillage sur la peau, satisfaisant en terme de brillance.

## Patentansprüche

1. Zusammensetzung zum Schminken oder zur Pflege der Haut, umfassend eine Fettphase, umfassend:
- mindestens ein Harz mit einem zahlenmittleren Molekulargewicht kleiner gleich 10.000 g/mol, das aus Kohlenwasserstoffharzen und Mischungen davon ausgewählt ist,
- mindestens ein Kohlenwasserstoff-Blockcopolymer, das aus gegebenenfalls hydrierten Blockcopolymeren mit mindestens einem Styrolblock und mindestens einem Block, der aus Butadien, Ethylen, Propylen, Butylen, Isopren oder einer Mischung davon ausgewählte Einheiten umfasst, ausgewählt ist,
- mindestens eine Fettsubstanz mit einer Schmelztemperatur von mehr als 25°C, die aus Wachsen und pastösen Fettsubstanzen ausgewählt ist,
wobei die Zusammensetzung kein flüchtiges Öl umfasst oder weniger als 5 Gew.-% oder besser weniger als 2 Gew.-% flüchtiges Öl, bezogen auf das Gesamtgewicht der Zusammensetzung, enthält,
- mindestens ein nichtflüchtiges Öl,
und wobei die Zusammensetzung bei Umgebungstemperatur (25°C) in flüssiger Form vorliegt.

2. Zusammensetzung nach Anspruch 1, **dadurch gekennzeichnet, dass** sie wasserfrei ist oder weniger als 10 Gew.-% Wasser oder besser weniger als 4%, bezogen auf das Gesamtgewicht der Zusammensetzung, umfasst.

3. Zusammensetzung nach einem der vorhergehenden Ansprüche, **dadurch gekennzeichnet, dass** sie keine Silikonverbindung umfasst oder weniger als 10 Gew.-% Silikonverbindung oder besser weniger als 4%, bezogen auf das Gesamtgewicht der Zusammensetzung, umfasst.

4. Zusammensetzung nach einem der vorhergehenden Ansprüche, **dadurch gekennzeichnet, dass** das Harz ein zahlenmittleres Molekulargewicht im Bereich von 250 bis 10.000 g/mol aufweist.

5. Zusammensetzung nach einem der vorhergehenden Ansprüche, **dadurch gekennzeichnet, dass** das Harz ein zahlenmittleres Molekulargewicht kleiner gleich 5000 g/mol, insbesondere im Bereich von 250 bis 5000 g/mol, vorzugsweise kleiner gleich 2000 g/mol, insbesondere im Bereich von 250 bis 2000 g/mol, und bevorzugt kleiner gleich 1000 g/mol, insbesondere im Bereich von 250 bis 1000 g/mol, aufweist.

6. Zusammensetzung nach einem der vorhergehenden Ansprüche, **dadurch gekennzeichnet, dass** die Kohlenwasserstoffharze aus Inden-Kohlenwasserstoffharzen, aliphatischen Pentadienharzen, gemischten Pentadien-Inden-Harzen, Dienharzen von Cyclopentadiendimeren, Dienharzen von Isopropendimeren und Mischungen davon ausgewählt sind.

7. Zusammensetzung nach einem der vorhergehenden Ansprüche, **dadurch gekennzeichnet, dass** es sich bei dem Harz um ein Inden-Kohlenwasserstoffharz handelt, das sich aus der Polymerisation von Indenmonomer und Monomer, das aus Styrol, Methylinden, Methylstyrol und Mischungen davon ausgewählt ist, ergibt.

8. Zusammensetzung nach dem vorhergehenden Anspruch, **dadurch gekennzeichnet, dass** das Inden-Kohlenwasserstoffharz hydriert ist.

9. Zusammensetzung nach einem der Ansprüche 1 bis 8, **dadurch gekennzeichnet, dass** es sich bei dem Harz um ein Indenharz handelt, das aus hydrierten Inden/Methylstyrol/Styrol-Copolymeren ausgewählt ist.

10. Zusammensetzung nach einem der Ansprüche 1 bis 8, **dadurch gekennzeichnet, dass** es sich bei dem Harz um ein aliphatisches Pentadienharz handelt, das sich aus der Polymerisation des Monomers 1,3-Pentadien (trans- oder cis-Piperylen) und von Monomer, das aus Isopren, Buten, 2-Methyl-2-buten, Penten, 1,4-Pentadien und Mischungen davon ausgewählt ist, ergibt.

11. Zusammensetzung nach einem der Ansprüche 1 bis 8, **dadurch gekennzeichnet, dass** es sich bei dem Harz um ein gemischtes Pentadien-Inden-Harz handelt.

12. Zusammensetzung nach einem der Ansprüche 1 bis 8, **dadurch gekennzeichnet, dass** es sich bei dem Harz um ein Dienharz von Cyclopentadiendimeren handelt, das sich aus der Polymerisation eines ersten Monomers, das aus Inden und Styrol ausgewählt ist, und eines zweiten Monomers, das aus Cyclopentadienmonomeren ausgewählt ist, ergibt.

13. Zusammensetzung nach einem der Ansprüche 1 bis 8, **dadurch gekennzeichnet, dass** es sich bei dem Harz um ein Dienharz von Isoprendimeren handelt, das sich aus der Polymerisation mindestens eines Monomers, das aus α-Pinen, β-Pinen, Limonen und Mischungen davon ausgewählt ist, ergibt.

14. Zusammensetzung nach einem der vorhergehenden Ansprüche, **dadurch gekennzeichnet, dass** das Harz in einem Gehalt im Bereich von 0,1 bis 30 Gew.-%, bezogen auf das Gesamtgewicht der Zusammensetzung, vorzugsweise im Bereich von 0,3 bis 20 Gew.-%, weiter bevorzugt im Bereich von 0,5 bis 15 Gew.-%, vorliegt.

15. Zusammensetzung nach einem der vorhergehenden Ansprüche, **dadurch gekennzeichnet, dass** das Wachs aus
- Kohlenwasserstoffwachsen,
- durch katalytische Hydrierung von tierischen oder pflanzlichen Ölen mit linearen oder verzweigten C₈-C₃₂-Fettketten erhaltenen Wachsen,
- hydrierten Ölen, die bei 25°C fest sind,
- Silikonwachsen,
- fluorierten Wachsen
- und/oder Mischungen davon
ausgewählt ist.

16. Zusammensetzung nach einem der vorhergehenden Ansprüche, **dadurch gekennzeichnet, dass** die pastöse Fettsubstanz aus
- Lanolin und Derivaten davon,
- Polyolethern, die aus Ethern von Pentaerythrit und Polyalkylenglykol, Ethern von Fettalkohol und Zucker und Mischungen davon ausgewählt sind,
- polymeren oder nichtpolymeren Silikonverbindungen,
- polymeren oder nichtpolymeren fluorierten Verbindungen,
- Vinylpolymeren, insbesondere:
- fettlöslichen Polyethern, die sich aus der Polyveretherung zwischen einem oder mehreren C2-C100- und vorzugsweise C2-C50-Diolen ergeben,
- Estern
- und/oder Mischungen davon
ausgewählt ist.

17. Zusammensetzung nach einem der vorhergehenden Ansprüche, **dadurch gekennzeichnet, dass** die bei 25°C feste Fettsubstanz bzw. festen Fettsubstanzen in der Zusammensetzung in einem Gehalt im Bereich von 0,1 bis 20 Gew.-%, bezogen auf das Gesamtgewicht der Zusammensetzung, vorzugsweise im Bereich von 0,5 bis 15 Gew.-%, vorliegen.

18. Zusammensetzung nach dem vorhergehenden Anspruch, **dadurch gekennzeichnet, dass** das nichtflüchtige Öl in der Zusammensetzung in einem Gesamtgehalt im Bereich von 10 bis 90 Gew.-%, bezogen auf das Gesamtgewicht der Zusammensetzung, vorzugsweise im Bereich von 10 bis 80 Gew.-%, vorliegt.

19. Zusammensetzung nach einem der vorhergehenden Ansprüche, **dadurch gekennzeichnet, dass** die Fettphase der Zusammensetzung in der Zusammensetzung in einem Gesamtgehalt im Bereich von 10 bis 95 Gew.-%, bezogen auf das Gesamtgewicht der Zusammensetzung, vorzugsweise im Bereich von 15 bis 85 Gew.-%, vorliegt.

20. Zusammensetzung nach einem der vorhergehenden Ansprüche, **dadurch gekennzeichnet, dass** es sich bei dem Kohlenwasserstoff-Blockcopolymer um ein gegebenenfalls hydriertes Copolymer mit Styrolblöcken und Ethylen/C₃₋₄-Alkylen-Blöcken handelt.

21. Zusammensetzung nach dem vorhergehenden Anspruch, **dadurch gekennzeichnet, dass** das Kohlenwasserstoff-Blockcopolymer aus gegebenenfalls hydrierten Styrol-Ethylen/Propylen-, Styrol-Ethylen/Butadien- und Styrol-Ethylen/Butylen-Diblockcopolymeren und gegebenenfalls hydrierten Styrol-Ethylen/Butadien-Styrol-, Styrol-Butylen/Ethylen-Styrol-, Styrol-Isopren-Styrol- und Styrol-Butadien-Styrol-Triblockcopolymeren ausgewählt ist.

22. Zusammensetzung nach einem der Ansprüche 20 und 21, **dadurch gekennzeichnet, dass** es sich bei dem Kohlenwasserstoff-Blockcopolymer um eine Mischung von hydriertem Styrol-Butylen/Ethylen-Styrol-Triblockcopolymer und Styrol-Ethylen/Butylen-Diblockcopolymer handelt.

23. Zusammensetzung nach einem der Ansprüche 20 bis 22, **dadurch gekennzeichnet, dass** das Kohlenwasserstoff-Blockcopolymer in der Zusammensetzung in einem Gehalt im Bereich von 0,1 bis 15 Gew.-%, bezogen auf das Gesamtgewicht der Zusammensetzung, vorzugsweise im Bereich von 0,5 bis 10 Gew.-% und weiter bevorzugt im Bereich von 1 bis 8 Gew.-% vorliegt.

24. Zusammensetzung nach einem der vorhergehenden Ansprüche, **dadurch gekennzeichnet, dass** das Gewichtsverhältnis von Harz zu Kohlenwasserstoff-Blockcopolymer im Bereich von 1/1 bis 4/1 liegt.

25. Zusammensetzung nach einem der vorhergehenden Ansprüche, **dadurch gekennzeichnet, dass** die Zusammensetzung mindestens eine pulverförmige Phase, die aus Pigmenten, Perlmutten und Mischungen davon ausgewählt ist, umfasst.

26. Zusammensetzung nach einem der vorhergehenden Ansprüche, **dadurch gekennzeichnet, dass** die Zusammensetzung eine wässrige Phase in einem Gehalt im Bereich von 3 bis 80 Gew.-%, bezogen auf das Gesamtgewicht der Zusammensetzung, vorzugsweise im Bereich von 5 bis 60 Gew.-%, umfasst.

27. Zusammensetzung nach einem der vorhergehenden Ansprüche, **dadurch gekennzeichnet, dass** die Zusammensetzung einen kosmetischen Bestandteil, der aus Antioxidantien, Duftstoffen, Konservierungsmitteln, Neutralisationsmitteln, Tensiden, Sonnenschutzmitteln, Vitaminen, Feuchtigkeitsspendern, Selbstbräunungsverbindungen, Antifaltenmitteln, Emollientien, hydrophilen oder lipophilen Wirkstoffen, Radikalfängern, Sequestriermitteln, Desodorantien, Filmbildnern und Mischungen davon ausgewählt ist, umfasst.

28. Nichttherapeutisches Verfahren zum Schminken oder zur Pflege der Haut, bei dem man auf die Haut eine Zusammensetzung nach einem der Ansprüche 1 bis 27 aufbringt.

29. Verwendung einer Zusammensetzung nach einem der Ansprüche 1 bis 27 zum Erhalt einer Abscheidung, insbesondere eines Make-ups auf der Haut, die hinsichtlich Glanz zufriedenstellend ist.

## Claims

1. Composition for make-up or care of the skin containing a fatty phase containing:
- at least one resin of number average molecular weight less than or equal to 10,000 g/mol, selected from hydrocarbon resins and mixtures thereof,
- at least one hydrocarbon block copolymer selected from the block copolymers, which may be hydrogenated, containing at least one styrene block and at least one block containing units selected from butadiene, ethylene, propylene, butylene, isoprene or one of the mixtures thereof,
- at least one fatty substance whose melting point is greater than 25°C selected from waxes and pasty fatty substances,
the said composition containing no volatile oil, or containing less than 5% by weight, or better less than 2% by weight of volatile oil relative to the total weight of the composition,
- at least one nonvolatile oil,
and the said composition being in liquid form at ambient temperature (25°C).

2. Composition according to Claim 1, **characterised in that** it is anhydrous, or **in that** it contains less than 10% of water by weight or better less than 4%, relative to the total weight of the composition.

3. Composition according to any one of the previous claims, **characterised in that** it contains no silicone compound, or **in that** it contains less than 10% by weight of silicone compound or better less than 4%, relative to the total weight of the composition.

4. Composition according to any one of the previous claims, **characterised in that** the resin displays a number average molecular weight ranging from 250 to 10,000 g/mol.

5. Composition according to the previous claim, **characterised in that** the resin displays a number average molecular weight less than or equal to 5000 g/mol, in particular ranging from 250 to 5000 g/mol, preferably less than or equal to 2000, in particular ranging from 250 to 2000 g/mol and preferably less than or equal to 1000 g/mol, in particular ranging from 250 to 1000 g/mol.

6. Composition according to any one of the previous claims, **characterised in that** the hydrocarbon resins are selected from indene hydrocarbon resins, aliphatic pentadiene resins, mixed pentadiene and indene resins, diene resins of cyclopentadiene dimers, diene resins of isoprene dimers and mixtures thereof.

7. Composition according to any one of the previous claims, **characterised in that** the resin is an indene hydrocarbon resin derived from the polymerisation of an indene monomer and a monomer selected from styrene, methylindene, methylstyrene and mixtures thereof.

8. Composition according to the previous claim, **characterised in that** the indene hydrocarbon resin is hydrogenated.

9. Composition according to any one of Claims 1 to 8, **characterised in that** the resin is an indene resin selected from the hydrogenated indene/methylstyrene/styrene copolymers.

10. Composition according to any one of Claims 1 to 8, **characterised in that** the resin is an aliphatic pentadiene resin derived from the polymerisation of the monomer 1,3-pentadiene (trans or cis piperylene) and a monomer selected from isoprene, butene, 2-methyl-2-butene, pentene, 1,4-pentadiene and mixtures thereof.

11. Composition according to any one of Claims 1 to 8, **characterised in that** the resin is a mixed pentadiene and indene resin.

12. Composition according to any one of Claims 1 to 8, **characterised in that** the resin is a diene resin of cyclopentadiene dimers derived from the polymerisation of a first monomer selected from indene and styrene, and a second monomer selected from the dimers of cyclopentadiene.

13. Composition according to any one of Claims 1 to 8, **characterised in that** the resin is a diene resin of isoprene dimers derived from the polymerisation of at least one monomer selected from α-pinene, β-pinene, limonene and mixtures thereof.

14. Composition according to any one of the previous claims, **characterised in that** the resin is present at a level ranging from 0.1 to 30% by weight, relative to the total weight of the composition, preferably ranging from 0.3 to 20% by weight, more preferably ranging from 0.5 to 15% by weight.

15. Composition according to any one of the previous claims, **characterised in that** the said wax is selected from:
- hydrocarbon waxes,
- waxes obtained by catalytic hydrogenation of animal or plant oils having linear or branched C₈-C₃₂ fatty chains,
- hydrogenated oils set at 25°C,
- silicone waxes,
- fluorinated waxes,
- and/or mixtures thereof.

16. Composition according to any one of the previous claims, **characterised in that** the said pasty fatty substance is selected from:
- lanoline and derivatives thereof
- polyol ethers selected from ethers of pentaerythritol and polyalkylene glycol, ethers of fatty alcohol and sugar, and mixtures thereof.
- silicone compounds whether or not polymeric
- fluorinated compounds whether or not polymeric
- vinyl polymers, in particular:
- liposoluble polyethers resulting from polyetherification between one or more C2-C100, preferably C2-C50, diols,
- esters,
- and/or mixtures thereof.

17. Composition according to any one of the previous claims, **characterised in that** the said fatty substance or substances solid at 25°C are present in the composition at a level ranging from 0.1 to 20% by weight, relative to the total weight of the composition, preferably ranging from 0.5 to 15% by weight.

18. Composition according to the previous claim, **characterised in that** the said nonvolatile oil is present in the composition at a total level ranging from 10% to 90% by weight, relative to the total weight of the composition, preferably ranging from 10% to 80% by weight.

19. Composition according to any one of the previous claims, **characterised in that** the fatty phase of the composition is present in the composition at a total level ranging from 10% to 95% by weight, relative to the total weight of the composition, preferably ranging from 15% to 85% by weight.

20. Composition according to any one of the previous claims, **characterised in that** the hydrocarbon block copolymer is a copolymer, which may be hydrogenated, with styrene blocks and C3-C4 ethylene/alkylene blocks.

21. Composition according to the previous claim, **characterised in that** the hydrocarbon block copolymer is selected from diblock copolymers, which may be hydrogenated, of styrene-ethylene/propylene, styrene-ethylene/butadiene, styrene-ethylene/butylene and triblock copolymers, which may be hydrogenated, of styrene-ethylene/butadiene-styrene, styrene-butylene/ethylene-styrene, styrene-isoprene-styrene and styrene-butadiene-styrene.

22. Composition according to either one of Claims 20 and 21, **characterised in that** the hydrocarbon block copolymer is a mixture of a hydrogenated styrene-butylene/ethylene-styrene triblock copolymer and a styrene-ethylene/butylene diblock copolymer.

23. Composition according to any one of Claims 20 to 22, **characterised in that** the hydrocarbon block copolymer is present in the composition at a level ranging from 0.1% to 15% by weight, relative to the total weight of the composition, preferably ranging from 0.5% to 10% by weight, and more preferably ranging from 1% to 8% by weight.

24. Composition according to any one of the previous claims, **characterised in that** the ratio by weight of the resin to the hydrocarbon block copolymer ranges from 1/1 to 4/1.

25. Composition according to any one of the previous claims, **characterised in that** the composition contains at least one powder phase selected from pigments, nacres, and mixtures thereof.

26. Composition according to any one of the previous claims, **characterised in that** the composition contains an aqueous phase at a level ranging from 3% to 80% by weight, preferably ranging from 5 to 60% by weight, relative to the total weight of the composition.

27. Composition according to any one of the previous claims, **characterised in that** the composition contains a cosmetic ingredient selected from antioxidants, perfumes, preservatives, neutralising agents, surfactants, sunscreens, vitamins, hydrating agents, self-tanning compounds, antiwrinkle active substances, emollients, hydrophilic or lipophilic active substances, anti-free radical agents, sequestering agents, deodorants, filmogenic agents, and mixtures thereof.

28. Non-therapeutic procedure for make-up or care of the skin comprising the application onto the skin of a composition according to any one of Claims 1 to 27.

29. Use of a composition according to any one of Claims 1 to 27, to obtain a deposit, in particular make-up, on the skin, which is satisfactory in terms of sheen.
